(19) **Europäisches Patentamt**
**European Patent Office**
**Office européen des brevets**

(11) **EP 2 393 443 B1**

(12) **EUROPÄISCHE PATENTSCHRIFT**

(45) Veröffentlichungstag und Bekanntmachung des Hinweises auf die Patenterteilung:
**08.08.2018 Patentblatt 2018/32**

(21) Anmeldenummer: **10702599.1**

(22) Anmeldetag: **25.01.2010**

(51) Int Cl.:
***A61B 18/12*** *(2006.01)*

(86) Internationale Anmeldenummer:
**PCT/EP2010/000419**

(87) Internationale Veröffentlichungsnummer:
**WO 2010/089037 (12.08.2010 Gazette 2010/32)**

(54) **HF-CHIRURGIEEINRICHTUNG**

HF SURGERY DEVICE

DISPOSITIF POUR CHIRURGIE HF

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **06.02.2009 DE 102009007861**
**09.03.2009 DE 102009012387**

(43) Veröffentlichungstag der Anmeldung:
**14.12.2011 Patentblatt 2011/50**

(73) Patentinhaber: **Erbe Elektromedizin GmbH**
**72072 Tübingen (DE)**

(72) Erfinder:
• **EISELE, Florian**
**79108 Freiburg (DE)**

• **SCHALL, Heiko**
**72622 Nürtingen (DE)**

(74) Vertreter: **Rüger, Barthelt & Abel**
**Patentanwälte**
**Webergasse 3**
**73728 Esslingen (DE)**

(56) Entgegenhaltungen:
**EP-A1- 1 112 720**  **WO-A1-01/12089**
**WO-A1-2005/117735**  **WO-A1-2008/101356**
**DE-A1-102005 007 769**  **US-A1- 2006 200 120**
**US-A1- 2007 255 269**

**Beschreibung**

Die Erfindung betrifft eine HF-Chirurgieeinrichtung, umfassend mindestens zwei HF- Generatoren nach dem Oberbegriff des Patentanspruches 1.
Es ist eine Vielzahl von HF-chirurgischen Geräten bekannt, die im Allgemeinen aus einem HF-Generator und einem Instrument bestehen. Sehr viele HF-chirurgische Instrumente sind als so genannte monopolare Instrumente ausgebildet, bei denen am Patienten eine großflächige indifferente Elektrode (meist am Oberschenkel) mit gutem Kontakt zum Körper befestigt wird, so dass der eigentliche Behandlungsstrom von einer Behandlungselektrode zum Koagulieren oder Schneiden oder dergleichen HF-chirurgischen Behandlung durch den Körper und die indifferente Elektrode fließt.
Bei manchen Operationen wird nun gewünscht, dass mehrere Operateure an ein und demselben Patienten gleichzeitig mit HF-chirurgischen Instrumenten arbeiten. Aus der DE 10 2005 007 769 A1 ist eine HF-Chirurgieeinrichtung bekannt, bei welcher mehrere Instrumente jeweils über einen Schalter an einem einzigen Ausgang eines einzigen HF-Generators angeschlossen werden können. Zwar ist mit einer derartigen HF-Chirurgieeinrichtung ein gleichzeitiges Operieren möglich, jedoch können die Instrumente immer nur im selben Betriebsmodus betrieben werden, der vom HF- Generator vorgegeben ist. Weiterhin ist bei der bekannten Anordnung ein Problem darin zu sehen, dass die Instrumente bzw. Aktivelektroden am Ausgang des HF-Generators über Relais parallel angeschlossen werden. Sofern beide Operateure gleichzeitig arbeiten, speist der Generator beide Aktivelektroden parallel. Die vom HF- Generator abgegebene Leistung teilt sich jedoch ungleichmäßig gemäß den Gewebeeigenschaften an den Aktivelektroden auf. Eine Regelung der eingestellten Ströme, wie sie bei HF-Generatoren üblich ist, kann auf diese Weise nicht mehr durchgeführt werden. Um nun zu gewährleisten, dass zwei Instrumente mit verschiedenen Einstellungen des HF-Generators arbeiten können, ist es denkbar, zwei vollständig voneinander unabhängige HF-Chirurgieeinrichtungen jeweils mit einer eigenen Aktiv- und einer eigenen Neutralelektrode zu betreiben. In diesem Fall sind zwar vollständig unabhängige Leistungs- und Betriebsarteneinstellungen realisierbar. Bei ungünstiger Anordnung der Neutralelektroden, insbesondere dann, wenn ein Körpergebiet von beiden HF-Strömen gleichzeitig durchflossen wird, können jedoch neuromuskuläre Stimulationen durch Schwebungseffekte auftreten. Aus diesem Grund wird diese Vorgehensweise als gefährdend angesehen.
Aus der WO 2008/101356 A1 ist eine HF-Chirurgieeinrichtung bekannt, die einen einzigen Generator aufweist. Dieser ist über einen Umschalter mit mehreren Instrumenten verbunden, die durch diesen alternativ zugleich oder auch sequentiell bestromt werden können. Dadurch kann ein höherer Strom an eine Einzelelektrode geliefert werden und die Instrumente können besser individuell geregelt werden. WO 2005/117735 A1 offenbart zwei HF-Generatoren, die Behandlungsströme an zwei verschiedene Elektrodepaare abgeben.

Aufgabe der Erfindung ist es, eine HF-Chirurgieeinrichtung der eingangs genannten Art dahin gehend weiter zu bilden, dass eine neuromuskuläre Stimulation vermieden, die HF-chirurgischen Instrumente aber dennoch in verschiedenen Betriebsarten betrieben werden können. Diese Aufgabe wird durch eine HF-Chirurgieeinrichtung nach Patentanspruch 1 gelöst.
Insbesondere wird die Aufgabe durch eine HF-Chirurgieeinrichtung gelöst, die mindestens zwei HF-Generatoren, mindestens einen Neutralelektrodenausgang für eine Neutralelektrode, mindestens zwei Ausgänge zum Anschluss von HF- chirurgischen Instrumenten zum Beaufschlagen der HF-chirurgischen Instrumente mit Behandlungsströmen und Umschalteinrichtungen aufweist. Hierbei sind für jedes HF-chirurgische Instrument ein gesonderter HF-Generator und für alle Instrumente bzw. HF-Generatoren eine einzige Neutralelektrodenanordnung vorgesehen. Die Umschalteinrichtung ist mit den HF-Generatoren in einer derart steuernden Verbindung angeordnet, dass die HF-Generatoren nie gleichzeitig, also ausschließlich nacheinander die Behandlungsströme erzeugen und an die Instrumente abgeben.

Es werden also gemäß der Erfindung zwei verschiedene HF-chirurgische Generatoren vorgesehen, die jeweils in einen Betriebsmodus geschaltet werden können, der dem zu verwendenden HF-chirurgischen Instrument entspricht. Regelungsvorgänge können ebenfalls durchgeführt werden, da jeder Generator einzeln oder alleine betrieben wird, wenn auch immer nur für kurze Zeit.

Die Umschalteinrichtung kann als gesonderte Einheit vorgesehen werden. Vorzugsweise umfasst jedoch die Umschalteinrichtung eine steuernde Einheit oder einen Master-Generator, welche die anderen HF-Generatoren als Slave-Generatoren über SYNC-Impulse oder dergleichen steuernde Impulse steuert. Auf diese Weise kann die gesamte HF-Chirurgieeinrichtung kompakt aufgebaut werden. Insbesondere soll hierbei der Master-Generator einen der eingangs genannten HF-Generatoren umfassen.

Die Umschalteinrichtung ist vorzugsweise derart ausgebildet, dass die Anzahl von mit ihr verbundenen HF-Generatoren festgestellt und deren Steuerung entsprechend der festgestellten Anzahl in einer gleichmäßigen Reihenfolge erfolgt. Auf diese Weise ist es dem Operationsteam möglich, ohne gesonderte manuelle Einstellung der Gerätschaften mit einem, zwei oder mehr HF-Generatoren (und HF-chirurgischen Instrumenten) zu arbeiten.

Die Umschalteinrichtung und die HF-Generatoren werden mit Kommunikationsschnittstellen, zum Beispiel einem Feldbus ausgestattet und derart ausgebildet, dass den HF-Generatoren definierte Beginn-Zeitpunkte und/oder Zeitfenster ($T_{On1/2}$) mitgeteilt werden, ab denen und/oder während denen die HF-Generatoren einen Behandlungsstrom erzeugen. Diese Zeitfenster werden nun vorzugsweise derart eingestellt, dass eine hinreichende Dauer für jeden HF-Generator zur Verfügung steht, so dass dieser ein behandlungstechnisch wirksames Signal abgibt und auch regeln kann.

Die Umschalteinrichtung ist vorzugsweise derart ausgebildet, dass zwischen dem Abgeben von Behandlungsströmen der HF-Generatoren Pausen liegen. Dadurch wird gewährleistet, dass die Schaltungen ausschwingen können, also keine Restströme eines Stromabgabevorganges mit dem Beginn eines darauf folgenden Stromabgabevorganges überlappen. Die Vorteile dieses Verfahrens sind weiter oben bereits beschrieben.

Vorzugsweise beträgt die Taktfrequenz 10 kHz bis 100 kHz. Auf diese Weise wird gewährleistet, dass einerseits eine quasi-kontinuierliche Operation erfolgen kann, ohne dass der Operateur einen wesentlichen Unterschied zum Arbeiten mit einem einzigen HF-Generator (und angeschlossenem einzigen HF-chirurgischen Instrument) bemerkt. Andererseits ist aber dennoch der Betrieb mehrerer Generatoren und HF-chirurgischer Instrumente möglich.

Bevorzugte Ausführungsformen der Erfindung ergeben sich aus den nachfolgenden Ausführungsbeispielen der Erfindung, die anhand von Abbildungen näher erläutert werden. Hierbei zeigen

- Fig. 1 Ein stark schematisiertes Blockschaltbild einer Ausführungsform der erfindungsgemäßen HF-Chirurgieeinrichtung,

- Fig. 2 eine erste Möglichkeit der Steuerung von zwei HF-Chirurgiegeneratoren anhand von Ausgangssignalen und

- Fig. 3 eine zweite Ausführungsform einer Steuerung von mehreren HF-Chirurgiegeneratoren.

In der nachfolgenden Beschreibung werden für gleiche und gleich wirkende Teile dieselben Bezugsziffern verwendet.

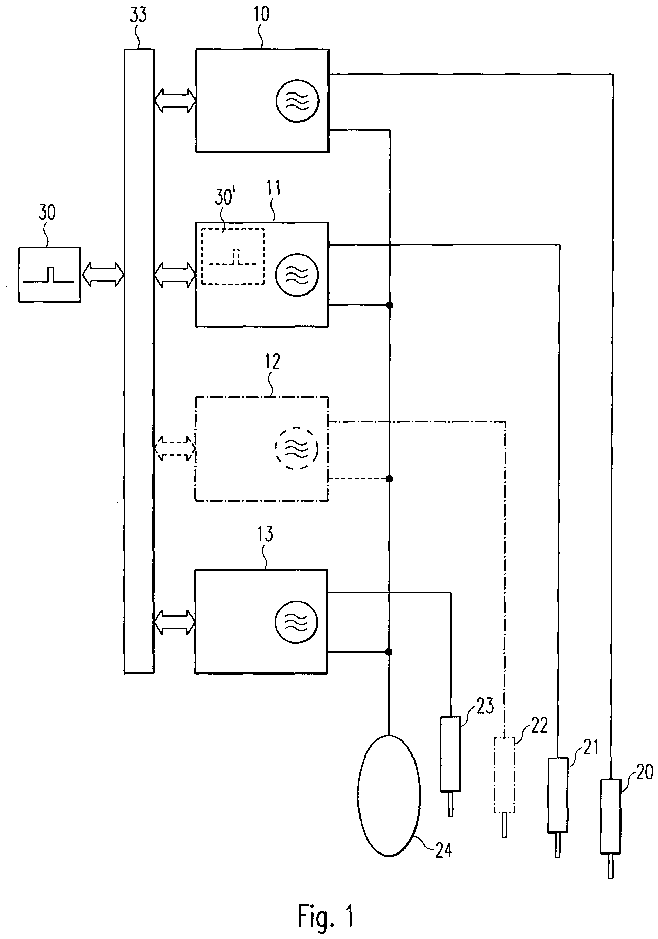

Gemäß Fig. 1 ist eine Vielzahl von HF-Generatoren 10, 11, 12 und 13 vorgesehen. Die Ausgänge dieser HF-Generatoren 10 bis 13, die im Normalfall jeweils mit einer Neutralelektrode 24 verbunden werden, sind nun zusammengeschaltet und liegen an dieser einzigen Neutralelektrode 24. Die weiteren Ausgänge der HF-Generatoren 10 bis 13 sind mit HF-chirurgischen Instrumenten 20, 21, 22 und 23 verbunden, die hier nur sehr stark schematisiert angedeutet sind. Die punktierten Linien der Darstellung des HF-Generators 12 und des dazu gehörigen HF-chirurgischen Instrumentes 22 sollen andeuten, dass die Anzahl der HF-Generatoren im Wesentlichen beliebig ist.

Alle HF-Generatoren 10 bis 13 stehen über einen gemeinsamen Bus 33 miteinander sowie mit einer Umschalteinrichtung 30 in Verbindung. Diese Umschalteinrichtung 30 kann auch in einem der Generatoren als Umschalteinrichtung 30' (hier HF-Generator 11) angeordnet sein. Der hier so benannte Bus 33 hat insbesondere die Funktion, eine Synchronisation der HF-Generatoren vorzunehmen, die bekanntlich derart gesteuert werden können, dass sie ihr HF-Signal (typischerweise 300 kHz bis 4 MHz) in extrem kurzen Zeiten an und abschalten können.

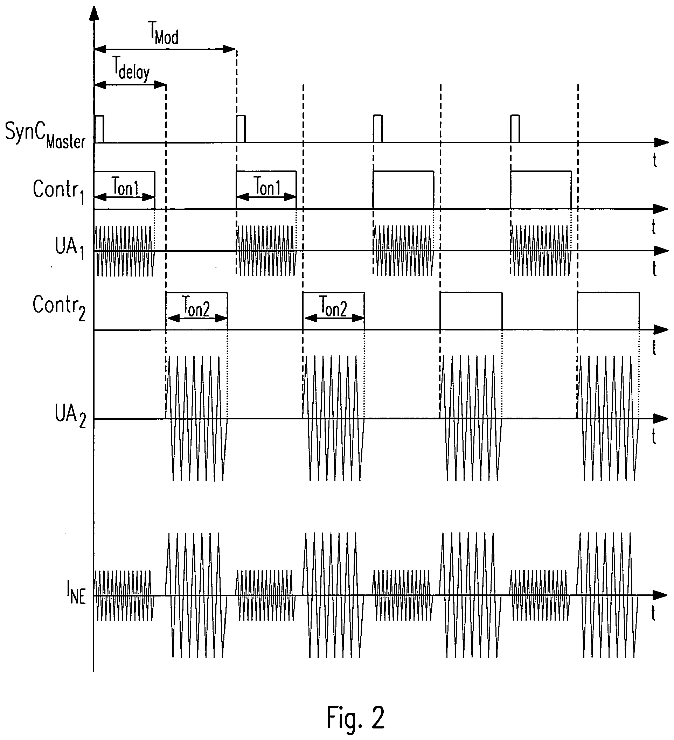

In Fig. 2 ist nun ein Zeitdiagramm aufgezeigt, das die Verwendung von lediglich zwei HF-Generatoren zeigt.

Die Umschalteinrichtung 30 (oder 30') gibt eine Pulsfolge $SYNC_{Master}$ ab, die mit einer Periodendauer $T_{Mod}$ wiederholt wird. Weiterhin wird von der Umschalteinrichtung (30, 30') dem zweiten angeschlossenen Generator eine Verzögerungszeit $T_{delay}$ sowie Einschaltzeiten $T_{Onl}$ und Ton2 mitgeteilt. Dadurch wird folgend auf jeden $SYNC_{Master}$ ein Steuersignal Contn abgegeben, das den ersten HF-Generator 10 derart ansteuert, dass dieser für die Zeit $T_{Onl}$ ein Ausgangssignal $UA_1$ abgibt. Nach der Verzögerungszeit $T_{delay}$ wird ein zweites Steuersignal Contr2 erzeugt, währenddessen (Ton2) der zweite Generator ein Ausgangssignal $UA_2$ abgibt. Dadurch fließt ein Strom $I_{NE}$ durch die Neutralelektrode, der in Fig. 2 im untersten Diagramm gezeigt ist und sich aus den beiden Teilströmen $UA_1$ und $UA_2$ zusammensetzt. Als Regel muss hierbei gelten, dass $T_{delay}$ größer ist als $T_{On1}$ und $T_{delay} + T_{On2}$ kleiner ist als $T_{Mod}$.

Aus Fig. 2 ist auch ersichtlich, dass die Zeiträume derart gewählt sind, dass die Ausgangsspannungen $UA_1$ und $UA_2$ jeweils abgeklungen sind, bevor die nächste Ausgangsspannung wieder ansteigt.

Auf diese Weise werden den beiden Generatoren innerhalb der Modulation jeweils "Zeitscheiben" $T_{On1}$ und $T_{On2}$ zugeteilt, die über das Signal $SYNC_{Master}$ zeitlich synchronisiert werden. Die Zeitscheiben dürfen sich nicht überlappen, damit es im Strom durch die gemeinsame Neutralelektrode zu keinen Schwebungsströmen - und damit neuromuskulären Stimulationen - kommen kann.

Beide Generatoren können nun in unterschiedlichen Betriebsarten und Leistungseinstellungen betrieben werden, da sie unabhängig voneinander geregelt und aktiviert werden.

Beschränkungen hinsichtlich der Betriebsartenauswahl sind natürlich auch vorhanden. So zum Beispiel sind kontinuierliche Betriebsarten der Generatoren ohne Modulation nicht erlaubt. Die Summe der relativen Aktivierungsdauern (duty-cycle) muss kleiner 1 sein, wobei die relative Aktivierungsdauer T definiert ist zu:

$$\tau_{1,2} = T_{On1,2}/T_{Mod}$$

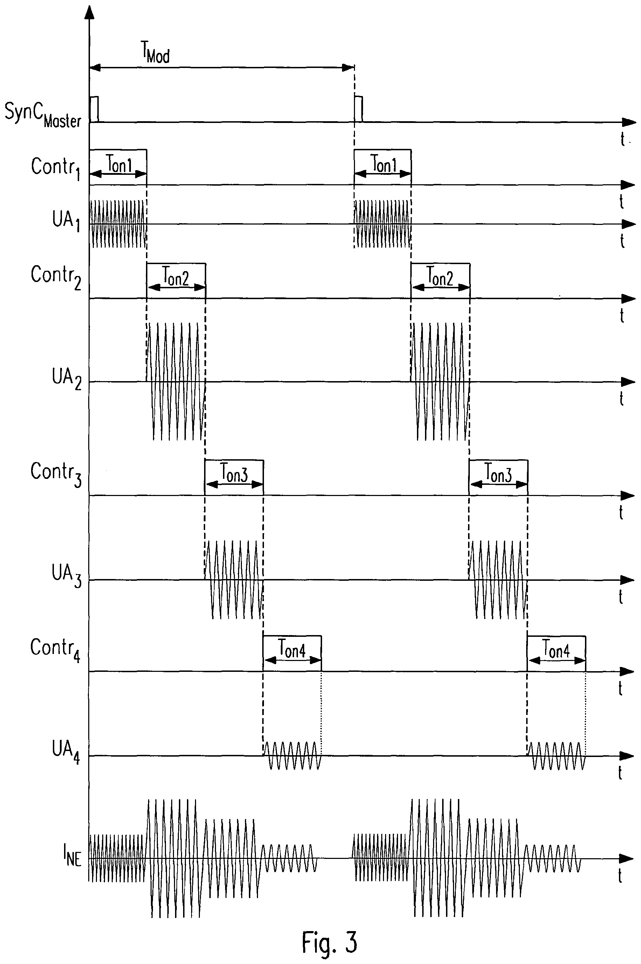

Bei der in Fig. 3 gezeigten Betriebsweise steuert der $SYNC_{Master}$-Impuls lediglich den ersten Generator derart, dass er während seiner Einschaltdauer $T_1$ ein Signal abgibt. Der weitere HF-Generator wird nun zur Abgabe eines Ausgangssignals $UA_2$ dann angesteuert, wenn die Ansteuerungszeit $T_{On1}$ des ersten Generators vorbei ist. Der dritte Generator wird vom zweiten Generator ebenso angesteuert und daraufhin auch der vierte Generator. Dann erst wieder erfolgt eine Ansteuerung durch den Impuls SYNCMaster nach der Periodendauer $T_{Mod}$. Der sich ergebende Strom $I_{NE}$ durch die indifferente Elektrode ist wieder in Fig. 3 ganz unten aufgezeichnet. Der wesentliche Unterschied dieser Ausführungsform gegenüber der nach Fig. 2 liegt also darin, dass die HF-Generatoren gewissermaßen als Kette nacheinander angesteuert werden und nur der erste Generator durch eine übergeordnete Pulsfolge angesteuert wird.

Aus Obigem ist ersichtlich, dass das erfindungsgemäße Verfahren des wechselweisen Ansteuerns der HF-Generatoren auf verschiedene Weise erfolgen kann.

Bezugszeichenliste

10 Generator
11 Generator
12 Generator
13 Generator
20 HF-chirurgisches Instrument
21 HF-chirurgisches Instrument
22 HF-chirurgisches Instrument
23 HF-chirurgisches Instrument
24 Neutralelektrode
30 Umschalteinrichtung
30' Umschalteinrichtung
33 Bus

**Patentansprüche**

1. HF-Chirurgieeinrichtung, umfassend

- mindestens zwei gesonderte HF-Generatoren (10 - 13) für mindestens zwei HF-chirurgische Instrumente (20-23);
- mindestens einen Neutralelektrodenausgang für eine Neutralelektrode (24);
- -mindestens zwei Ausgänge zum Anschluss der HF-chirurgischen Instrumente (20 - 23) zum Beaufschlagen der chirurgischen Instrumente mit Behandlungsströmen und
- Umschalteinrichtungen (30),

**dadurch gekennzeichnet,**
**dass** für jedes HF-chirurgische Instrument (20 - 23) ein gesonderter HF-Generator (10 - 13) und für alle HF-chirurgischen Instrumente (20 - 23) bzw. Generatoren (10 - 13) ein einziger Neutralelektrodenausgang vorgesehen ist,
**dass** die Umschalteinrichtung (30, 30') mit den HF-Generatoren (10 - 13) in einer derart steuernden Verbindung angeordnet ist, dass die HF-Generatoren (10 - 13) nie gleichzeitig sondern ausschließlich nacheinander die Behandlungsströme erzeugen und an die mindestens zwei HF-chirurgischen Instrumente (20 - 23) abgeben.

2. HF-Chirurgieeinrichtung nach Anspruch 1, **dadurch gekennzeichnet dass** die Umschalteinrichtung eine steuernde Einheit oder einen Master-Generator umfasst, welche die anderen HF-Generatoren (10 - 13) als Slave-Generatoren über SYNC-Impulse oder dergleichen steuernde Impulse steuert.

3. HF-Chirurgieeinrichtung nach einem der vorhergehenden Ansprüche, insbesondere nach Anspruch 2, **dadurch gekennzeichnet dass** der Master-Generator einen der HF-Generatoren (11) umfasst.

4. HF-Chirurgieeinrichtung nach einem der vorhergehenden Ansprüche, **dadurch gekennzeichnet dass** die Umschalteinrichtung derart ausgebildet ist, dass die Anzahl von mit ihr verbundenen HF-Generatoren (10 - 13) festgestellt und deren Steuerung entsprechend der festgestellten Anzahl in einer gleichmäßigen Reihenfolge erfolgt.

5. HF-Chirurgieeinrichtung nach einem der vorhergehenden Ansprüche, **dadurch gekennzeichnet dass** die Umschalteinrichtung (30, 30') und die HF-Generatoren (10 - 13) Kommunikationsschnittstellen, z.B. einen Feldbus (33) umfassen und derart ausgebildet sind, dass den HF-Generatoren (10 - 13) definierte Beginnzeitpunkte und/oder Zeitfenster ($T_{On1/2}$) mitgeteilt werden, ab denen und/oder während denen die HF-Generatoren (10 - 13) einen Behandlungsstrom erzeugen.

6. HF-Chirurgieeinrichtung nach einem der vorhergehenden Ansprüche, **dadurch gekennzeichnet dass** die Umschalteinrichtung (30, 30') derart ausgebildet ist, dass zwischen dem Abgeben von Behandlungsströmen der HF-Generatoren (10 - 13) Pausen liegen.

**Claims**

1. HF surgery device comprising:

- at least two separate HF generators (10 - 13) for at least two HF surgical instruments (20 - 23);
- at least one neutral electrode output for a neutral electrode (24);
- at least two outputs for connection of the HF surgical instruments (20 - 23) for loading the surgical instruments with treatment currents, and
- changeover devices (30),

**characterised in that**
a separate HF generator (10 - 13) is provided for each HF surgical instrument (20 - 23), and a single neutral electrode output is provided for all HF surgical instruments (20 - 23) and generators (10 - 13), the changeover device (30, 30') is arranged in a controlling connection to the HF generators (10 - 13) such that the HF generators (10 - 13) never simultaneously, but exclusively consecutively, generate the treatment currents and output these to the at least two HF surgical instruments (20 - 23) .

2. HF surgery device according to claim 1, **characterised in that** the changeover device comprises a controlling unit or a master generator which controls the other HF generators (10 - 13) as slave generators via SYNC pulses or similar controlling pulses.

3. HF surgery device according to one of the preceding claims, in particular claim 2, **characterised in that** the master generator comprises one of the HF generators (11).

4. HF surgery device according to any of the preceding claims, **characterised in that** the changeover device is configured such that the number of HF generators (10 - 13) connected thereto is established and they are controlled according to the established number in an even sequence.

5. HF surgery device according to any of the preceding claims, **characterised in that** the changeover device (30, 30') and the HF generators (10 - 13) comprise communication interfaces, e.g. a fieldbus (33), and are configured such that the HF generators (10 - 13) are given a defined starting time and/or time window ($T_{On1/2}$) from which and/or during which the HF generators (10 - 13) generate a treatment current.

6. HF surgery device according to any of the preceding claims, **characterised in that** the changeover device (30, 30') is configured such that pauses are present between the output of treatment currents from the HF generators (10 - 13).

**Revendications**

1. Dispositif pour chirurgie HF, comprenant :

- au moins deux générateurs HF (10 à 13) distincts pour au moins deux instruments de chirurgie HF (20 à 23) ;
- au moins une sortie d'électrode neutre pour une électrode neutre (24) ;
- au moins deux sorties pour la connexion des instruments de chirurgie HF (20 à 23) pour alimenter les instruments de chirurgie avec des courants de traitement, et
- des dispositifs de commutation (30),

**caractérisé en ce que**
pour chaque instrument de chirurgie HF (20 à 23), il est prévu un générateur HF (10 à 13) distinct, et pour tous les instruments de chirurgie HF (20 à 23) et/ou les générateurs (10 à 13), il est prévu une sortie d'électrode neutre unique,
le dispositif de commutation (30, 30') est monté avec les générateurs HF (10 à 13) dans une liaison de commande telle que les générateurs HF (10 à 13) ne produisent jamais en même temps les courants de traitement, mais exclusivement les uns après les autres, et les transmettent aux instruments de chirurgie HF (20 à 23), au nombre d'au moins deux.

2. Dispositif pour chirurgie HF selon la revendication 1, **caractérisé en ce que** le dispositif de commutation comprend une unité de commande ou un générateur pilote, qui commande les autres générateurs HF (10 à 13) en tant que générateurs asservis, à l'aide d'impulsions SYNC ou d'impulsions de commande analogues.

3. Dispositif pour chirurgie HF selon une des revendications précédentes, en particulier selon la revendication 2, **caractérisé en ce que** le générateur pilote comprend l'un des générateurs HF (11).

4. Dispositif pour chirurgie HF selon une des revendications précédentes, **caractérisé en ce que** le dispositif de commutation est agencé de manière telle que le nombre de générateurs HF (10 à 13) qui y sont reliés soit déterminé et leur commande s'effectue en fonction du nombre déterminé, dans un ordre régulier.

5. Dispositif pour chirurgie HF selon une des revendications précédentes, **caractérisé en ce que** le dispositif de commutation (30, 30') et les générateurs HF (10 à 13) comprennent des interfaces de communication, par exemple un bus de terrain (33), et sont agencés de manière telle que les générateurs HF (10 à 13) reçoivent des informations concernant des instants de début et/ou des fenêtres de temps définis ($T_{on1/2}$), à partir desquels et/ou pendant lesquelles les générateurs HF (10 à 13) produisent un courant de traitement.

6. Dispositif pour chirurgie HF selon une des revendications précédentes, **caractérisé en ce que** le dispositif de commutation (30, 30') est agencé de manière à ce qu'il y ait des pauses entre l'émission de courants de traitement des générateurs HF (10 à 13).

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005007769 A1
- WO 2008101356 A1
- WO 2005117735 A1